# EUROPEAN PATENT SPECIFICATION

(11) **EP 2 561 858 B1**
(45) Date of publication and mention of the grant of the patent: **06.04.2016**
(21) Application number: 12179425.9
(22) Date of filing: 06.08.2012
(51) Int. Cl.: A61K 8/63, A61K 8/64, A61K 8/97, A61Q 7/00, A61K 36/73, A61K 36/48

(54) **A composition for activating hair follicle stem cells to stimulate hair growth**
Zusammensetzung zur Aktivierung von Haarfollikelstammzellen zur Stimulierung des Haarwachstums
Composition destinée à activer des cellules souches de follicules pileux pour stimuler la croissance des cheveux

(30) Priority: 05.08.2011 CH 13042011
(43) Date of publication of application: 27.02.2013
(73) Proprietor: LABO Cosprophar AG, 4052 Basel (CH)
(72) Inventor:
(74) Representative: Coppo, Alessandro

(56) References cited:
- EP-A1- 1 859 774
- FR-A1- 2 951 944

## Description

### FIELD OF THE INVENTION

The present invention relates to a composition for activating hair follicle stem cells to stimulate hair growth.

The present invention originates in the trichological sector and, in particular, in the field of preparations for topical use to stimulate physiological hair regrowth.

In particular, the present invention relates to a trichological lotion for topical use, which acts on the human hair follicle stem cells to stimulate physiological hair regrowth in areas affected by thinning and/or baldness.

### BACKGROUND OF THE INVENTION

It is commonly known that the life cycle of hair envisages three different, sequential phases, defined anagen (growth), catagen (involution) and telogen (rest). The hair growth period is followed by a regression phase, in the course of which the deepest part of the follicle will meet programmed cell death.

Growth of the hair and the life cycle of the hair follicle are regulated by stem cells of the follicle itself, located in a permanent portion thereof, known as the bulge. Bulge stem cells are of small dimensions and are in a state of cell dormancy, which is the state of rest of the cell, otherwise knows as phase G0 of the cell cycle (Garaz L.A. Cotsarelis et al, The Journal of Clinical Investigation, 2011).

Bulge cells were the first hair follicle cells to be identified. Their capacity to form the hair follicle, the interfollicular epidermis and the sebaceous glands was subsequently demonstrated (Oshima *et al,* 2001; Costsarelis, 2006). Bulge zone cells demonstrate maximum growth capacity in vitro compared to cells from other hair follicle and epidermal regions. Cell fate experiments carried out using the impulse marking of nucleotides has revealed that bulge cells give rise to inferior follicles, including the outer root sheath, the matrix and the medulla (Ito *et al,* 2004). The fluorescence marking of bulge cells, using the K15 promoter activity, has revealed that the cells derived from the bulge area are found in all types of epithelial cells of the new inferior hair follicle and of the hair shaft, and are also present in the epidermis and in the sebaceous glands (Liu *et al,* 2003).

Roth *et al,* (2005) confirmed that the bulge area of the human hair follicle contains keratinocyte stem cells, while the hair matrix represents a cell compartment known as Transit Amplification (TA), in course of proliferation and differentiation.

Lyle *et al* (1998) demonstrated that human follicles in telogen and in anagen express the Cytokeratin-15 KET15 protein and high levels of integrin β1 in the basal layer of cells that respectively surround the bulb in telegen (secondary hair germ) and in the anagen region. Since the entire epithelium of the inferior follicle, beneath the isthmus, degenerates during the catagen hair phase of the hair cycle, the follicles in telogen constitute a rich source of stem cells. The keratinocytes of the matrix situated at the bottom of the hair bulb in anagen represent the TA cells, which differentiate, through rapid proliferation, into epithelial layers of the hair follicle.

It is also known that the follicles enter the anagen phase when chemical signals from the dermal papilla activate the dormant stem cells in the follicle bulge in telogen inciting them to proliferate, thus determining a cell generation that will produced the new hair. These cells are transit amplification cells or progenitor cells of the follicle matrix that will produce the new hair.

Bulge cells normally have a very slow cyclicity but can be stimulated/activated, as mentioned, by the dermal papilla to then move towards proliferation (Ryan R. *et al* 2011; Roh C. *et al* 2004).

In the anagen phase, the dermal papilla generates chemical signals that activate and instruct the bulge stem cells, which form the hair matrix by migrating to the base of the follicle. With this "migration", the bulge stem cells create a cell "path" that will give rise to the outer root sheath or ORS.

In response to further signals from the dermal papilla, the matrix cells, which derive from the stem cells, proliferate and commence n the differentiation process, moving upwards to form the stem and the inner sheath of the hair follicle.

The start of catagen is characterised by the cessation of cell proliferation and by the apoptosis of the matrix cells. During catagen the dermal papilla migrates towards the lowest portion of the bulge. This close proximity of the papilla to the bulge is thought to be essential for the commencement of another hair cycle. This allows interaction/activation of the resting bulge cells and a new hair growth cycle. At the time of catagen/telogen transition, some bulge cells migrate to meet the papilla, generating the hair germ.

Hair in telogen contains a population of cells at its base, which is in fact known as hair germ, situated in close proximity to the dermal papilla. The hair germ is activated to proliferate towards the end of the telegen phase, even before the bulge, to form, by surrounding the papilla, the matrix of the new bulb.

The cells of the germ, deriving from the bulge stem cells, and the bulge cells thus have the intrinsic capacity to proliferate and are directly involved in the initial anagen, contributing to different follicle compartment; they are however dormant in the telogen phase.

A link between baldness and a hair follicle stem cell functionality defect has been recently established. In particular, according to an American study published in the Journal of Clinical Investigation in January 2011, in androgenic alopecia, which represents the most common form of baldness, instead of being active, the follicle stem cells present on the scalp, are as if switched off, "asleep". According to researchers at the University of Pennsylvania, including George Cotsarelis, inactive follicle stem cells are unable to transform into mature cells, the so-called progenitor cells.

In individuals affected by androgenic alopecia (AGA), the follicles that form at the start of the new anagen phase decrease in size with the passing of time (miniaturisation), leading, as time proceeds, to the formation of a hair that is smaller than the previous one. The result is the formation of a microscopic hair. Garza A.L., Cotsarelis *et al* (2011) discovered that androgenic alopecia is characterised by the failure of hair follicle stem cells to convert into progenitor cells. So as to arrive at these conclusions, George Cotsarelis and colleagues analysed samples of scalp affected and not affected by baldness extracted from 54 mean aged between 40 and 65 years. It emerged from the comparison that the progenitor cells were markedly depleted in the follicles of volunteers with a bald scalp, compared to volunteers with non-bald tissue. The researchers concluded that the baldness derives from a stem cell activation problem rather than from the number of stem cells present in the follicles when the conversion into progenitor cells in the scalp must commence.

Nevertheless, the fact that there are also a normal number of stem cells in the bald scalp makes the idea of 'reactivating the cells' and identifying new topical treatments against androgenic alopecia possible.

From the study of the global gene expression of bulge cells, it has been seen that these cells express the intermediate filament protein Keratin-15 (KRT15), while the expression of CD34 is limited to the cells that are to be found immediately below the bulge area and that possess the phenotype of a progenitor cell subject to cell apoptosis at the end of the anagen phase.

High levels of KRT15 expression are clearly linked to the phenotype of a stem cell (dormancy and small dimension properties). KRT15 therefore represents a stem cell activity marker.

In their study, Garza A.L. *et al* isolated single-cell suspensions of epithelial scalp cells of bald and non-bald volunteers, collected from the same individuals subject to AGA.

From determination of KRT15 by means of antibodies there is revealed the presence of high levels of this marker for stem cells both in the bald and non-bald scalp, thus supporting the hypothesis that follicle stem cells are maintained in the bald scalp.

From determination of CD34 by means of antibodies there is revealed how the number of positive CD34-positive follicle cells (progenitor cells) in anagen, which are located next to the bulge in the outer root heath, have decreased by around 10 times in the bald scalp compared to the non-bald scalp.

Highlighting the loss of the activated bulge cells (progenitor cells) but not of the dormant bulge cells (stem cells) in the individuals subject to AGA confirms the hypothesis that AGA results from the lesser conversion of follicle stem cells into progenitor cells.

Scalp follicles atrophy, however a reserve of stem cells remains that convert into progenitor cells but in a lesser measure than in the non-bald scalp.

A decrease of the cell population, possibly due to a lack of supply from the bulge cells, could potentially have as a consequence a reduced number of cells from the bulb matrix in the new lower follicle. Lower numbers of matrix cells would have as a consequence reduced hair shafts, since these cells are immediately responsible for hair shaft production.

Dermatologists classify alopecias by subdividing them into the cicatricial and noncicatricial categories. Some types of alopecia (e.g. ad es. lichen planopilaris, discoid lupus erythematous and graft-versus-host-disease) are associated with the destruction of bulge follicle stem cell destruction and with permanent hair loss. In the reversible types of alopecia (e.g. alopecia areata), the inflammation affects the follicle progenitor cells, but spares the stem cells thereof. In these diseases, regrowth takes place on elimination of the inflammation and on the consequent regeneration of the hair follicle starting from undamaged stem cells.

Most of the trichological preparations available on the market target the hair bulbs and act on the metabolism, nutrition, oxygenation and microcirculation of the scalp with the aim of increasing and accelerating hair growth.

The European patent application EP 1859774A1 discloses hair growth products containing corosolic acid. The corosolic acid may be incorporated in the product as such or in the form of extracts from Eriobotrya japonica and Lagerstroemia speciosa.

The French patent application FR 2951944 A1 discloses hair growth products containing a rice peptide hydrolysate from non-fermented rice which stimulates hair follicles. In the disclosed hair growth product 5-alpha reductase inhibitors may be also added.

Unlike the common research projects that having the aims as set out above, the Applicant has investigated the presence of scalp stem cells in the bulge area and of non-differentiated mother cells responsible for the birth of a new anagen phase inside the follicle and for the development and growth of a new hair.

One of the aims of the invention consists in providing a cosmetic composition to stimulate physiological hair growth in volunteers affected by baldness or thinning of the hair.

Another object of the invention consists in providing a composition for trichological use that is suitable for reactivating or keeping active the scalp stem cells of bald volunteers or of volunteers with thinning hair.

### SUMMARY OF THE INVENTION

The Applicant has found that follicle stem cells that are inactive or in a state of dormancy in the scalp can be reactivated by applying a trichological formulation that combines one component that is biologically active on the stem cells with at least one component that stimulates the proliferation of dermal papilla cells.

According to a first aspect of the present invention there is therefore provided a composition that is active on hair follicle stem cells to stimulate hair growth comprising the synergistic association of
i) one biologically active component that promotes the expression of marker proteins associated with the growth and proliferation of follicle stem cells, in an effective amount and
ii) one biologically active component that stimulates the proliferation of dermal papilla mesenchymal cells, in an effective amount and a cosmetically acceptable carrier,
wherein the biologically active component i) comprises a rice peptide extract and the biologically active component ii) comprises corosolic acid.

The composition of the invention acts on the follicle stem cell viability, reactivating the life cycle of even dormant hair follicles of the scalp in the thinning area and/or areas affected by baldness.

In addition, the composition of the invention promotes the expression of stem cell marker proteins, of kerating-15, alpha-6 integrin, beta-catenin, P63 in particular, which are associated with the growth and proliferation of follicle stem cells.

The composition of the invention then increases the viability of follicle stem cells keeps the stem cells from endogenous and exogenous damage, both in the anagen phase and during miniaturisation. Furthermore, the composition of the invention reactivates dormant stem cells of the scalp while regenerating the follicles and stimulating new hair growth. In particular, follicle regeneration requires activation of the stem cells located in the bulge region.

Within the scope of the invention, the term "rice extract" shall mean an extract based on peptides, polypeptides, biologically active peptide fraction, obtained by hydrolysis from the proteins of the grains of the rice plant.

Typically, when reference is made to "rice hydrolysed proteins", this shall also mean, without limitation, proteins present "in the rice peptide extract".

Plant for obtaining the peptide extract belong to the family Poaceae or Graminacee and to the genus Oryza, specifically Oryza sativa L.

Typically, the rice peptide extract comprises one or more of the following proteins:
- Albumin, water soluble, typically at 0.3-9.9% by weight compared to the total protein fraction,
- Globulin water insoluble, soluble in saline solutions, typically at 1.6-14.0% by weight compared to the total protein fraction,
- Glutelin, water, saline solution and alcohol insoluble, soluble in alkaline solutions, typically at 73-94% by weight compared to the total protein fraction,
- Prolamin, water insoluble, soluble in alcohol typically at 1.7-7.6% by weight compared to the total protein fraction,

Glutelins are a group of proteins that are particularly suitable for the applications of the invention. Of these, the Oryzenin is a preferred protein.

In certain embodiments, the active ingredient i) of the composition of the invention is an unfermented rice hydrolysed protein obtained by hydrolysis of the proteins of a grain of rice. According to these embodiments, fermentation is not performed until the proteins are degraded by the protease of the fermentation yeasts and the extract remains concentrated in native proteins.

Typically, the part of the rice plant used to obtain the protein fraction i) is the de-husked grain of rice, following a decortication phase.

In one embodiment, the protein fraction is hydrolysed into small peptides, the molecular weight of which is less than 5000 Da.

The component ii) of the composition of the invention is corosolic acid, a substance of plant origin that promotes the synthesis of growth factors in the dermal papilla, which prolong the anagen phase of the hair and increase follicle vascularisation.

Corosolic acid is present for example in the leaves of the Loquat (Eriobotrya japonica), a plant of the Rosaceae family that is also known as Japanese plum. The said Loquat leaves have properties that promote the in vitro growth of dermal papilla cells and keratinocytes. In addition, since the dimensions of the dermal papilla affect the thickness of the hair, the proliferation of papilla cells induced by the corosolic acid leads to the growth of thick, strong hair.

In some embodiments of the invention, a Loquat leaf extract is used as the biologically active component ii) as corosolic acid is present in the leaves. The extraction takes place by common extraction techniques. As an alternative to Loquat leaves, corosolic acid is obtainable from a Banaba leaf extract of the Lagerstroemia speciosa botanical species of the Lythraceae family.

In some embodiments, the composition of the invention further comprises a component iii) that inhibits 5-alpha-reductase. The composition according to these embodiments has the following three simultaneous actions on the scalp:
- action on hair follicle stem cells to promote the expression of those proteins/follicle stem cell markers, associated with regeneration of the hair,
- action of the dermal papilla to stimulate the proliferation of these cells and the relevant production of growth factors for the hair follicle,
- 5-alpha-reductase inhibition action to hinder follicle miniaturisation.

The component iii) has a trichological action since the testosterone, entering the cytoplasm of the follicle cells, is converted by the cytoplasmic 5alpha-reductase enzyme into its active form, dihydrotestosterone (DHT) that binds to the androgen receptors on the hair follicles interfering with the normal growth cycle. Specifically, a DHT-receptor complex is formed that is moved into the nucleus where the proteins that exercise specific biological action are synthesised through the gene transcription process,.

In hair follicles, the androgen receptors are located in the dermal papilla, not in the epithelial cells. This means that the dermal papilla is the main objective for the androgens in the hair follicle.

In some embodiments, said component iii) is an extract of Red clover or of Trifolium pratense, of the Leguminosae family.

Red clover is a perennial, herbaceous plant that grows spontaneously in temperate and subtropical regions and that grows both in fields and in mountain regions. The flowers are purple inflorescences. The inflorescences are gathered in summer when in full bloom.

The red clover flower head is particularly rich in Biochanin A and contains isoflavones such as genistein and daidzein typical of soya and derivatives thereof (formononetin).

Biochanin-A has an inhibiting effect on type I and II 5-alpha reductase. It therefore reduces the activity of the 5-alpha reductase enzyme, which transforms the hormone testosterone into dihydrotestosterone DHT, which causes follicle miniaturisation. It also reduces the inflammatory process of the follicle that characterises alopecia.

In particular, Biochanin-A is the isoflavone that is most representative of red clover. In some embodiments, Biochanin, in particular type A Biochanin, can be used as an alternative to the extract of Red clover. Suitable alternative sources of Biochanin-A are Baptisia tinctoria (Wild Indigo), Medicago sativa (Alfalfa), Sophora japonica (Japanese Pagoda Tree) and Vigna radiate (Mungbean).

In some embodiments, the composition of the invention can contain one or more additional active ingredients such as for example amino acids, vitamins, plant glycoproteins of the Lectin family, plant protein hydrolysates, trace elements (silicon, zinc, copper, etc.), DNA hydrolysate, RNA hydrolysate, salicylic acid, glycyrrhetinic acid, allantoin, microalgae extracts, algae extracts.

In some embodiments, the composition of the invention comprises the following biologically active components or substances:
- peptide rice extract, of 0.0001 to 10% by weight for example,
- corosolic acid or Trifolium pratense extract, of 0.0001 to 10% by weight for example,
- optionally, nicotinic acid ester, for example benzyl nicotinate, of 0.001 % to 1 % by weight for example

The composition of the inventions is used in the topical application and has proven effective in preventing and/or treating forms of baldness or of thinning of the hair, such as for example androgenic alopecia and telogen defluxion.

The composition for topical application of the invention can presented itself both in liquid form as a lotion, solution, solid or semi-solid suspension such as gel, cream, ointment, salve, mask and prolonged release transdermal patch.

The composition of the invention can be formulated in a form suitable for trichological uses and for topical application, as a solution or aqueous suspension, dispersion or gel for example.

In some embodiments, the carrier of the aqueous composition is water based and can contain an alcohol suitable for cosmetic applications such as ethyl alcohol and/or one or more cosmetically acceptable glycols such as propylene or pentylene or hexylene glycol and mixtures thereof.

In the form of solution or aqueous suspension or dispersion, the composition can generally contain from around 1-99.9% water. In some embodiments, water is present in an amount of between 5-95%. In other embodiments, water is present in an amount of between 10-90% by weight.

In the case of liquid formulation, the synergical active ingredients of the invention can be conveniently dissolved in a physiologically acceptable liquid such as water, alcohol, hydroalcoholic, glycerol solution and other liquids suitable for local application.

By way of example, the compositions of the invention in liquid form can be prepared by dissolving the water soluble components and the remaining components in alcohol to then reunite the various fractions under agitation. The resulting mixture can then be buffered to reach a pH interval conveniently selected between 5 and 7 so as to be compatible with the pH of the scalp to then be filtered and packaged in suitable containers such as bottles or vials.

In some embodiments, the trichological compositions of the invention can comprise excipients commonly utilised in the formulation of cosmetic preparations for local use, such as preservatives, bactericidal agents, stabilisers, emulsifiers, surface-active agents, buffers, humectants, dyes and other excipients commonly used in cosmetic / pharmaceutical preparation techniques.

The composition of the invention can be applied directly to the scalp in an effective amount.

For example, in treating androgenic alopecia, a cosmetically active amount of the lotion of the invention is applied directly to the scalp one or more times daily, conveniently for cycles having a duration of 2-3 months alternating with periods of rest.

Typically, the biologically active components in the composition of the invention are present in variable quantities, for example of between 0.001% by weight and 10% by weight, typically of between 0.1 % and 5% by weight.

According to another aspect of the invention, there is provided a cosmetic treatment method that included the local application, at the level of the scalp, of an effective amount of a composition of the previously-described type.

### DETAILED DESCRIPTION OF THE INVENTION

In some embodiments, the active ingredient i) of the composition of the invention is an unfermented rice hydrolysis protein obtained by hydrolysis of the proteins of grains of rice.

The protein hydrolysate, consisting of amino acids, peptides and proteins can be obtained by alkaline, acidic or enzymatic hydrolysis of the rice.

In some embodiments, obtaining the rice proteins envisages an initial removal of the liposoluble portion, after which follows the protein extraction by means of subsequent fractionation by taking advantage of the different solubilities of the protein and using appropriate solvents (Hamada J. S., 1997).

By way of example, one suitable method for extracting rice proteins is alkaline hydrolysis, followed by acid precipitation (Connor et al., 1977; Jiamyangyuen et al., 2005); the method envisages, as optimal extraction conditions, an alkaline environment with basic pH of around 11 for example and variable reaction times of between 10 minutes and 2 hours, of 45 minutes for example.

Following removal of the lipid portion, the rice sample is suspended in distilled water at a ration of 1:5 (1g in 5ml of water) for example, subsequently a portion of 5 ml for example of this suspension is adjusted to a basic pH of 11 (e.g. by means of a 0.2M solution of NaOH) for example. The reaction is conducted at a temperature greater than room temperature, for example 30°C, with reaction time of 45 min for example.

Following the reaction, the pH of the suspension is brought to around 7 with an acid solution, for example 0.2 M of HCl to block the reaction. The residue is separated from the soluble product by filtration.
- Lastly, the soluble portion is assayed to quantify the protein, amino acid and TOC (total organic carbon) content.

Enzymatic hydrolysis can be performed as an alternative to alkaline extraction. Certain problems that can manifest in condition of high alkalinity, such as the formation of undesirable reaction products (for example Lisynoalanine) can be overcome with this procedure,

In addition, such conditions can cause protein denaturation and hydrolysis, an increase in the Maillard reaction that leads to the appearance of dark-coloured products, an increase in the extraction on non-protein derivatives that coprecipitate with the proteins and reduce the quality of the isolated product.

Examples of enzymatic hydrolysis are described in Mass Production Method for Rice Protein Isolate and Nutritional Evaluation, Morita T., Kiriyama S., Journal of food Science, November 1993, Vol 58, Issue 6, 1393-96.

A typical example of extraction envisages that the rice flour be mixed with 0.6% of a Termamyl (alpha-amylase) solution at room temperature (23°C). The product obtained is heated at 97°C for 2 hours under continuous agitation. There follows a simultaneous gelation and liquefaction process.

The product obtained by filtration and washing with boiling water, defined RPI - Rice Protein Isolate - is characterised by more than 90% of pure protein (dry matter).

An alternative extraction method that can be used is described in Extraction of protein and amino acids from deoiled rice bran by subcritical water hydrolysis, Sereewatthanawut I., Prapintip S., Watchiraruji K., Goto M., Sasaki M., Shotipruk A., Bioresource Technology 99 (2008), 555-61.

This method envisages the production of a hydrolysis reaction in subcritical water, in a temperature range of between 100 and 220°C for 0-30min.

This method allows the extraction of a greater quantity of proteins than the conventional method in a basic environment and generally the yield increases the greater the temperature and the reaction time.

A further method for the extraction of rice proteins that can be used to obtain the component i) is described in Preparation and Functional Properties of Rice Bran Protein Isolate, Wang M., Hettiarachchy N. S., Qi M., Burks W., Siebenmorgen T., Journal of agricultural and food chemistry, 1999, 47, 411-16.

According to this method, a sample of defatted rice is suspended in distilled water at pH5 and treated with Phytase and Xylanase with an incubation phase of di 55°C for 2hr.

### BRIEF DESCRIPTSION OF THE DRAWINGS

The present invention will be described in detail hereunder and with reference to the drawings, wherein:
Figure 1 shows a graph which records the increases in expression of the Keratin-15 marker in ORS paraffin sections (ex-vivo),
Figure 2 illustrates a graph which records the increases in expression of the α-6 integrin marker in frozen ORS sections (ex-vivo),
Figure 3 illustrates a graph which records the increases in expression of the beta catenin marker in frozen ORS sections (ex-vivo),
Figure 4 illustrates a graph which records the increases in expression of P63 marker in frozen ORS sections (ex-vivo),
Figure 5 illustrates a graph which records the toxicological effects of a peptide rice extract on a hair biopsy after 20-day treatment,
Figure 6 illustrates a graph which summarised the results of in vitro tests of the component i) on human follicle stem cells,
Figure 7 illustrates a graph which records the effects of corosolic acid on hair follicle growth factors,
Figure 8 illustrates a graph which records the results of the 5-alpha reductase inhibition activity on the part of Biochanin-A.

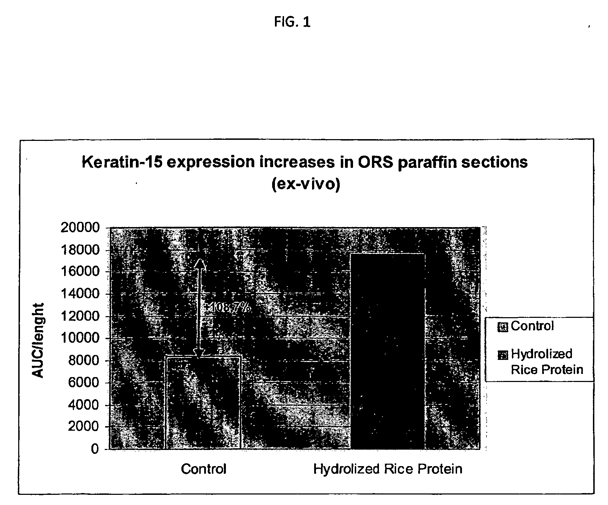

The markers indicated in Figures 1-4 are associated to the growth and proliferation of follicle stem cells and are reliable indicators of hair health. These markers are the Keratin K15, α6 integrin, β catenin and protein p63.

Specifically, Keratin-15 is am adult hair follicle stem cell marker, located in the bulge area and in the outer root sheath (ORS). The expression of Keratin-15 in stems cells leads to the synthesis of proteins known as keratins, which form the cellular cytoskeleton, typical of mature keratinocytes. Keratins comprise a multi-gene family of fibrous proteins that form the cytoskeleton of the cell. They can be classified on the basis of their molecular weight and of their iso-electric type I point (acid cytokeratins or with a low MW) and type II (basic cytokeratins or with a high MW).

In particular, Keratin -15 is widely recognised in numerous studies as a specific marker for the activity of hair follicle stem cells in the bulge.

The antibody used to determine Keratin-15 is the monoclonal C8/144B antibody. Bulge stem cells over-express the gene relating to Keratin-15.

Keratin-15 is widely expressed by bulge stem cells and only partially expressed in other parts of the outer root sheath (ORS): in proximity to the infundibulum and the lower part of the ORS.

The presence of Keratin-15 in these parts of the follicle also is due to the presence of cells migrated from the bulge, which weakly express this protein.

Integrins are integral membrane glycoproteins that principally mediate the bonding of the keratinocytes of the basal layer to the proteins of the extracellular matrix present in the basement membrane, but can also mediate intercellular (cell-cell) adhesion. Integrins regulate the cell cycle, differentiation and apoptosis. In particular, l'α6-integrin is a stem cell marker located in the basement membrane of the outer root sheath of the follicle.

Adult stem cells are located in small, specialised sites called "niches", a microenvironment that provides the stem cells with support, protection and signals that regulate the relationship between cellular self-replication and cellular differentiation. Integrins are more abundant in stem cells than the Transit Amplification (TA) cells, descendants of the stem cells destined for differentiation. Stem cells require a strong adherence one to the other and with the basement membrane to maintain themselves as stem cells or to maintain their position in the "niche", adherence guaranteed by the integrins.

Integrins thus appear to contribute to preserving a stem cell's identity.

In their study, Kloepper JE et al (2008) revealed that in addition to being present in the bulge region, stem cells are also located in other areas of the human follicle where the basement membrane is present and α-6-integrin is present in all parts of the outermost root sheath (ORS) and of the basement membrane (BM) of the entire hair follicle.

β-catenin is a multifunctional protein that acts on the nucleus as transcription factor and at the level of the plasmatic membrane as modulator of cell adhesion. β-catenin is expressed in particular in the outer root sheath of the follicle, plays an important role in the growth of the hair follicle and in cell differentiation. It is part of a complex of proteins that constitute the intercellular junction. Beta-catenins are necessary for regulation of cell growth, cell-cell adhesion and cell differentiation.

In the absence of a mitotic signal from the outside, β-catenin is seized in a complex formed by different protein sub-units comprising APC proteins, tumour suppressor proteins, the main function of which is to reduce β-catenin levels.

To this end, the β-catenin molecules that are destroyed phosphorylate, sending them to the proteasome. This mechanism, in synergy with the binding of β-catenin to E-cadherin, transmembrane protein which indirectly limits the translocation of beta-catenin to the nucleus, causes the presence of low levels of free β-catenin at cytosolic level.

When there is a cytoplasmic accumulation of β-catenin, since it has eluded the degradation mechanism, there is a translocation of the nucleus where the protein interacts with Tcf/LEF transcription factors activating the expression of various genes involved in cell migration and proliferation. The latter is incremented. β-catenin thus has a significant role in controlling cell proliferation and death. The formation of the hair follicle takes place during embryogenesis through specific interactions between the epithelial cells of the skin and the underlying dermis. Epithelial cells form a columnar thickening called *placode.*

A *mesenchymal condensate* is subsequently formed just under the placode. Further interactions between them induce the placode to fall into the underlying dermis and the elongated placode surrounds the dermal condensate, which becomes the *dermal papilla.*

Lastly, the epithelial cells proliferate and differentiate in the various layers thus generating the hair follicle.

Huelsken J et al (2001) demonstrated that β-catenin has a dual control in hair formation:
a. it plays an essential role in the formation of the hair placode during embryogenesis
b. it is indispensable in the differentiation of skin stem cells in adults. In the absence of β-catenin, stem cells are incapable of giving rise to hair keratinocytes and originate epidermal keratinocytes.

Skin stem cells therefore require the presence of beta-catenin in order to differentiate in the hair follicles. By in fact observing the stem cells of β-catenin mutant mice, Huelsken J et al (2001) verified their anomalous differentiation in epidermal cysts, demonstrating that β-catenin represents a specific signal for the formation of the hair follicle.

P63 is a marker expressed by the stem cells of the outer root sheath ORS and by the stem cells of the bulge in mature hair. It is indispensable for cellular proliferation and morphogenesis of the hair follicle.

Protein p63 is essential during embryogenesis, above all for the development of stratified epithelia and mesenchymal tissue. It is involved in the morphogenesis of the follicle in that it is required for the formation of the hair placode as the *p63-*/-mouse phenotype demonstrates.

The DeltaNp63 isoforms seem to be the principal isoforms involved during the first and crucial hair formation stages. Acting as transcription activator, it regulates the expression of some genes such as for example β-catenin, which is important for hair development and the absence of which results in the early blockage of hair follicle formation. Protein p63 plays an important role in the stem cell function.

Pellegrini et al (2001) identify protein p63 as an epithelial stem cell marker. In their study, skin stem cells and the respective transitions cells (TA) are isolated and then cultivated in vitro for the purposes of obtaining holoclones and paraclones. Holoclones are cultured epithelial cells with high proliferative capacity capable of almost exclusively giving rise to clones while maintaining unaltered the original stem cell characteristics. Paraclones are cultured epithelial cells capable of almost exclusively giving rise to secondary clones, which are all differentiated.

Protein p63 has abundant holoclones but no paraclones. p63 is therefore a stem cell marker.

In mature follicles it is observed how the expression of DeltaNp63 isoforms is restricted to the bulge and to the regions of the matrix where it plays an important role in maintaining the cells in stem form, in self-renewal and in balancing the proliferation and differentiation of matrix cells.

The biologically active component i) of the composition of the invention is active and promotes the expression of the markers/proteins associated with the growth and proliferation of the previously described follicle stem cells.

On the other hand, the biologically active component ii) containing corosolic acid acts on the proliferation of the mesenchymal cells of the dermal papilla.

In particular, RT-PCR analysis carried out to research the cytokines involved in the hair cycle, shows that Loquat leaves and Corosolic Acid in particular, positively regulate the levels of expression in mRNA of FGF-7 (Fibroblast Growth Factor) VEGF (Vascular Endothelial Growth Factor) while the negatively regulate the expression of FGF-5 (Fibroblast Growth Factor-5) in dermal papilla cells. VEGF plays an important role in the growth cycle of hair following the increased vascularisation. FGF-5 stops hair growth by inhibiting the anagen process and promoting the transition of the hair from anagen to catagen. These results highlight how the effect of the plant extract of Loquat on hair growth is mediated by the regulation of growth factors in dermal papilla cells.

The present invention will now be described with reference to the following examples, which are provided for illustrative purposes only and are not to be construed as limiting the present invention.

### EXAMPLE 1

The activity of rice peptide extract (or peptide hydrolysate) i) of the composition of the invention on stem cell surface markers of the hair follicle, was demonstrated with ex-vivo studies on 6 mm scalp biopsies, obtained by punch biopsy.

### Materials and Methods

The scalp sections have a thickness of 4 microns, for the paraffin-embedded biopsies, and 6 microns, for the frozen biopsies. On average 5 follicles were examined for each biopsy in duplicate.

The active ingredient was applied locally as a 1 % solution once daily for 2 days. The evaluation was made by immunofluorescence: the markers that appear in green are evaluated; the nuclei of the cells are coloured in blue.

Quantification of immunoreactivity is carried out in accordance with the publications of Paus R. and is based on the average of 3 measurements of three separate rectangular sections in the images taken through Image Pro version VI software. The histograms generated were integrated to obtain an area with underlying curve. The measurements are normalised through the measurement of the length, the area or the number of cells, on the basis of the experiment for each section of the follicle.

The percentage increase values emerge from the total follicle to control ratio.

### Results

Keratin-15 expression increases in the outer epithelial sheath sections in the paraffin-embedded biopsies. There is a 108.7% increase compared to the control, as illustrated in Fig. 1.

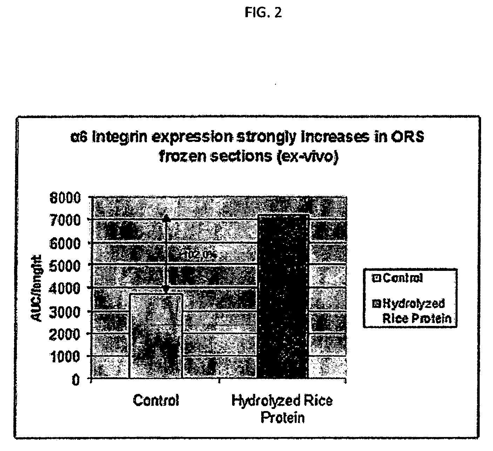

α-6 integrin expression increases in the outer epithelial sheath sections of the frozen biopsies. There is a 102.0% increase compared to the control, as illustrated in Fig. 2.

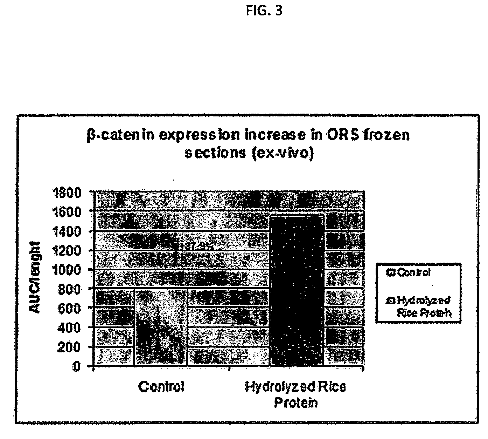

β-catenin expression increases in the outer epithelial sheath sections of the frozen biopsies. There is an 87.9% increase compared to the control, as illustrated in Fig. 3.

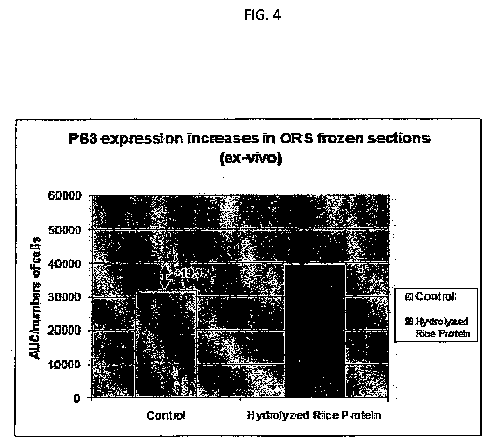

P63 expression increases in the outer epithelial sheath sections of the frozen biopsies. There is a 19.3% increase compared to the control, as illustrated in Fig. 4.

From the experiments carried out it therefore emerges that the biologically active component i) identified by the Applicant as Rice Hydrolysed Proteins (or peptide hydrolysate), contributes to improving the follicle stem cell markers associated with hair regeneration. Specifically, the component i) of the composition contributes:
1. to improving the expression of Keratin-15: protein involved in the initial phase of the differentiation path of the keratinocytes and capable of marking up to the least differentiated adult follicle cells relating to the bulge area.
2. to increasing the expression of α-6 integrin: one of the hair follicle stem cell markers, expressed at basal level of the cell matrix surrounding the dermal papilla and the ORS sheath.
3. to improving the expression of β-catenin, which plays a key role in hair follicle growth and differentiation.
4. to improving the expression of P63, protein linked to the capacity to generate hair follicles.

### EXAMPLE 2

The biologically active component i) was also tested for its capacity to positively interfere with hair growth. The study was carried out on 6 mm scalp biopsies obtained by punch biopsy containing 3 to 6 hairs, in duplicate. The active ingredient was applied topically as a 1 % aqueous solution every day, for 21 days. Photographs of the said biopsy were taken after 10, 14 and 21 days of cultivation using VivaCam.

The length of each hair is measured by means of software Image - Pro Analyzer and 3 to 6 hairs were measured, in duplicate.

The ex-vivo study on hair growth revealed how the active ingredient, used at 1%, increases hair elongation vs. the control:
after 10 days +65% ; - after 14 days + 148%; - after 21 days + 91%.

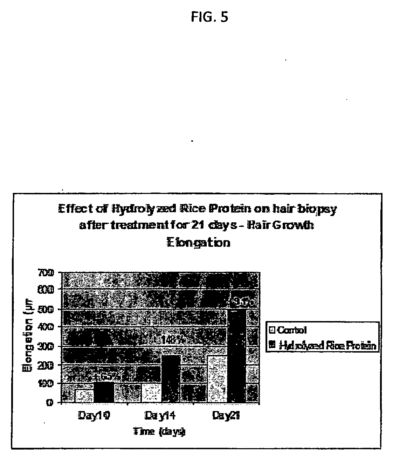

The data obtained is summarised in the accompanying Fig. 5.

The activity of the active ingredient on hair follicle stem cells in anagen therefore also translates into hair elongation.

### EXAMPLE 3

The activity of the active ingredient i) on human hair follicle stem cells was evaluated by means of an in-vitro experiment described hereunder.

In the course of life, human hair follicles are impoverished of stem cells, both following chronological ageing and following various types of stress, so much so that both the number of stem cells and their renewal activity decreases. In order to test the activity of the active ingredient on the life of the stem cells, human hair follicle stem cell cultures were created and apoptosis, i.e. programmed cell death, was quantified.

When the apoptosis process is activated the cell enzymes digest proteins and nucleic acids inside the cell, which destructures into small blisters.

UV radiations induce human cells to activate the programmed apoptosis mechanism, so that harmful mutations are not perpetuated.

The human hair follicle stem cells being studied were irradiated with UV rays, to reproduce a significant stress in the laboratory, and were treated with the compound dissolved in culture medium (modified DMEM medium) for 2 hr before and 20 hr after UV irradiation.

The irradiator is equipped with two monochrome UVB tubes (312nm, 15 W) and a radiometer (HD2302 LightMeter, Delta OHM).

The human hair follicle stems cells were irradiated with UVB 25mJ/cm² rays. Apoptosis was then quantified following irradiation, through quantification of specific enzymes (caspases), which are activated in the apoptosis phase, in particular caspases 3 and 7. The effector caspases 3 and 7 are specific proteases that are activated in the apoptosis phase. The apoptotic cells are identified under the microscope using fluorescent dyes.

After just 24 hours, the trial revealed a 10% reduction in cell apoptosis compared to the untreated control when evaluated at 005% of active ingredient.

That is to say, after just 24 hours the human hair follicle stem cells, under UV stress, begin to recover their efficiency.

Considering the results it is possible to hypothesise that the tested principle, "Rice hydrolysed proteins" exercises a protective action in respect of DNA damage in hair follicle stem cells.

The results are of particular interest since the action described is exhibited in just 24 hr of incubation.

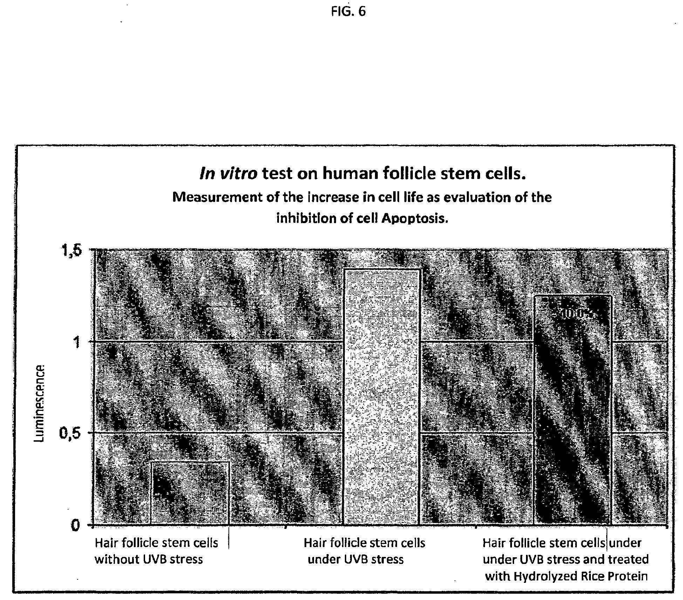

The data obtained is summarised in Fig. 6.

This Figure highlights that:
1. the hair follicle stems cells not under UVB stress rays are at the end of their lifespan,
2. The hair follicle stem cells under UVB stress rays reduce their lifespan by up to 3 times as much (303%) as the untreated group,
3. The hair follicle stem cells under UVB stress rays and treated for 24 hr with Rice Hydrolysed Protein increase their lifespan by 10%.

It follows that the biologically active component i), tested on human hair follicle stem cells is capable of generating the recovery of cell efficiency.

The images of the cell cultured confirm the results achieved, showing a reduced presence of apoptotic stem cells (green-positive) in percentage.

From analysis of the experimental data obtained it follows that the biologically active component i)
1. preserves the stem cell niche while ensuring a greater activity to the follicle for the purposes of regrowth.
2. guarantees a greater efficiency of the follicle stem cells after just 24 hours of product application.
3. activates the stem cells even in the follicles that have lost their regenerative capacity.
4. also activates the dormant stem cells in the thinning and bald areas.

### EXAMPLE 4

An in vitro experiment was carried out to evaluate the activity of the corosolic acid contained in a Loquat (Eriobotrya japonica) leaf plant extract, in particular when associated with rice hydrolysed protein i).

Human follicle dermal papilla cells were cultivated with different concentrations of Loquat/Japanese plum leaf extract for 4 days, on conclusion of which, the increase in cell proliferation compared to the control was evaluated by MTT Test. Results: +12% and +22% with 2.5 µg/ml and 10 µg/ml respectively.

In a further experiment, human follicle dermal papilla cells were incubated with different concentrations of Japanese plum extract for 2 hours, on conclusion of which the total RNA was isolated and the content of the mRNA was evaluated by RT-PCR.

When the cells were treated with 2.5 µg/ml of extract, the levels of mRNA expression of FGF-7, VEGF and BMP-2 increased significantly compared to the control: +30%, +38% and +47% (with 10 µg/ml of extract) respectively. While on the other hand, the level of mRNA expression of FGF-5 decreased compared to the control: - 20% with 2.5 µg/ml of extract.

This effect is significant since the FGF-7 and BMP-2 growth factors are capable of prolonging the anagen phase by means of the proliferation of follicle cells.

The test results prove that the Loquat/ Japanese plum leaf extract promotes the growth of hair while expressing FGF-7, BMP-2 (anagen prolongation) and VEGF (increase in vascularisation) and underexpressing FGF-5 (suppression of the anagen to catagen transition).

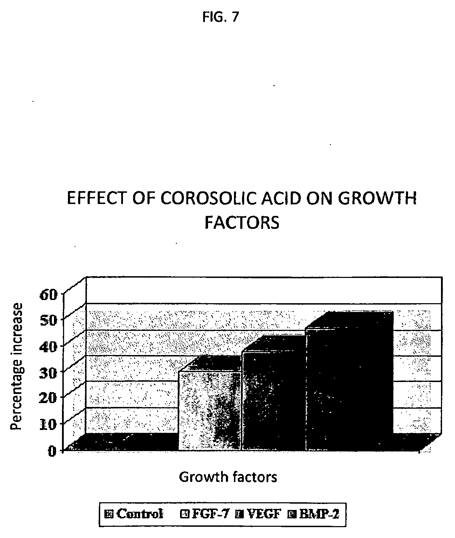

The results of the administration of corosolic acid on the growth factors of the follicle cells are provided in Fig. 7.

### EXAMPLE 5

The 5-alpha reductase inhibition capacity of the Biochanin, to which its trichological activity is correlated, is shown by an in vitro experiment.

In the experiment, skin fibroblasts, cells that contain different types of enzymes involved in the biosynthesis of steroids, were used. These cells represent a good system for the evaluation of the kinetic parameters of enzyme inhibitors such as 5-alpha reductase, delegated to steroid metabolism.

Fibroblasts were or were not incubated with 100µM of active Biochanin-A for 1 hour at 37°C before the addition of a known quantity of marked Testosterone, at the concentration of 1.5 micromoles. After 3 hr we proceeded with the extraction of the radioactive steroids. The amount of marked Testosterone and DHT are determined by TLC (Thin Layer Chromatography) to quantify 5-alpha reductase activity.

The results expressed as percentage inhibition of 5-alpha reductase activity show that the enzyme is significantly reduced in the presence of Biochanin-A
- 5-alpha reductase activity I: -64% compared to the negative control
- 5-alpha reductase activity II: -93% compared to the negative control.

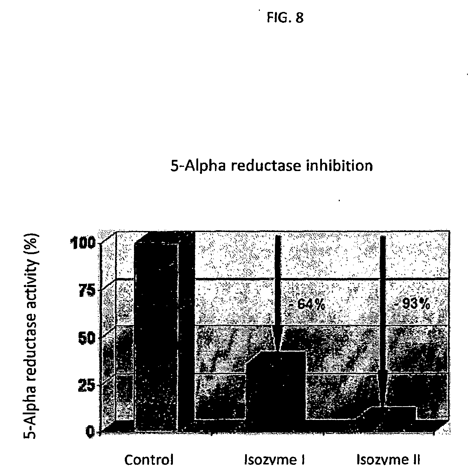

The results achieved are set out in the graph of Fig. 8.

### EXAMPLE 6

Cosmetic composition in vials for hair having the following formulation in % by weight

| | |
|---|---|
| Denatured alcohol | 51.6 |
| Water | q.s. 100 |
| Glycerine | 1.0 |
| Benzyl nicotinate | 0.12 |
| Plant glycoproteins | 0.01 |
| Lysine hydrochloride | 0.1 |
| Acetyl Cysteine | 0.08 |
| Bebzophenone-4 | 0.08 |
| Disodium Edta | 0.08 |
| Glycyrrhetinic acid | 0.05 |
| Menthol | 0.05 |
| Silicon | 0.04 |
| Glutamine | 0.04 |
| Adenosine | 0.01 |
| Allantoin | 0.01 |
| Biotin | 0.01 |
| Hematite extract | 0.008 |
| Zinc methionate | 0.01 |
| Rice Hydrolysed Protein | 0.1 |
| Soy Hydrolysed Protein | 0.005 |
| Hydrolysed Dna | 0.002 |
| Hydrolysed Rna | 0.002 |
| Pueraria Mirifica Root Extract | 0.1 |
| Eriobotrya Japonica Leaf Extract | 0.1 |
| Trifolium pratense lore extract | 0.1 |
| Salicylic acid | 0.001 |
| Glycogen | 0.001 |

### EXAMPLE 7

Cosmetic composition for hair lotion having the following formulation in % by weight.

| | |
|---|---|
| Denatured alcohol | 20.00 |
| Water | q.s. 100 |
| Benzyl nicotinate | 0.05 |
| Lysine hydrochloride | 0.01 |
| Plant glycoproteins | 0.01 |
| Acetyl cysteine | 0.05 |
| Menthol | 0.05 |
| Silicon | 0.01 |
| Zinc methionate | 0.04 |
| Biotin | 0.01 |
| Adenosine | 0.01 |
| Hematite extract | 0.008 |
| Hydrolysed Rice Protein* | 0.01 |
| Eriobotrya Japonica Leaf Extract** | 0.01 |
| Trifolium pratense flower extract | 0.01 |

### EXAMPLE 8

Cosmetic composition in the form of hair shampoo having the following formulation in % by weight.

| | |
|---|---|
| Water | Qs 100 |
| Sodium lauryl sulphate | 7.00 |
| Disodium Cocoamphodiacetate | 4.00 |
| Peg-18 Glyceryl Oleate/Cocoate | 1.4 |
| Glycerine | 1.00 |
| Glycol Distearate | 0.5 |
| Poliquaternium-10 | 0.3 |
| Propylene glycol | 0.3 |
| Steareth-4 | 0.3 |
| Peg-200 Hydrogenated Glyceryl Palmitate | 0.2 |
| Quaternium-80 | 0.13 |
| Lysine-HC1 | 0.1 |
| Cystein-HC1 | 0.1 |
| Peg-7 Glyceryl Cocoate | 0.08 |
| Hydrolysed keratin | 0.07 |
| Arginine | 0.003 |
| Rice Hydrolysed Protein | 0.001 |
| Eriobotrya Japonica Leaf Extract | 0.001 |
| Trifolium pratense flower extract | 0.001 |
| Dyes | |
| Fragrance | |
| Preservatives | |

### EXAMPLE 9

A randomised double-blind placebo-controlled in vivo clinical trial was carried out on a composition in lotion form according to one embodiment of the invention, to demonstrate the activity stimulating the physiological hair growth in volunteers with areas of the nape of the neck that are thinning or bald.

### 1.1. Scope of the study

The following trial has the purpose of evaluating the efficacy of the cosmetic lotion, suitable for the treatment of male androgenic alopecia.

A placebo-controlled clinical-instrumental study was carried out on 46 healthy male volunteers (23 volunteers for each treatment arm) presenting a cutaneous clinical picture characterised by grade II to IV androgenic alopecia. The efficacy of the test formulation was evaluated 2 to 4 months after treatment by means of the phototrichogram technique and the pull test technique. The instrumental analysis is completed by evaluation of the subjects participating in the study.

### 1.2 Study design

The study was carried out as follows: a) double blind, neither the study volunteer nor the investigator were aware of the allocation of the active and placebo treatments, b) placebo-controlled, the study was conducted by comparison with the results obtained in a group of volunteers using the placebo, c) randomised, the two arms, active and placebo treatment, were randomised according to an appropriate statistical procedure (Efron's biased coin design). Randomisation was performed by the Study Director.

### 1.3 Test formulation (test product)

The composition tested was a trichological lotion containing
i) rice peptide extract (hydrolysed rice proteins)
ii) corosolic acid from a Eriobotrya japonica plant extract having the following formulation by weight (w/w;

### Formulation of the test composition (Lotion 1)

| | |
|---|---|
| Denatured alcohol | 51.6 |
| Water | q.s. 100 |
| Glycerin | 1.0 |
| Butylene glycol | 0.2 |
| Plant glycoproteins | 0.01 |
| Lysine hydrochloride | 0.1 |
| Acetylcysteine | 0.08 |
| Benzophenone-4 | 0.08 |
| Disodium EDTA | 0.08 |
| Glycyrrhetinic acid | 0.05 |
| Menthol | 0.05 |
| Triethylamine | 0.03 |
| Glutamine | 0.04 |
| Adenosine | 0.01 |
| Allantoin | 0.01 |
| Biotin | 0.01 |
| Hematite extract | 0.008 |
| Zinc methionate | 0.01 |
| Hydrolysed Rice Protein | 0.11 |
| Hydrolysed Soy Protein | 0.005 |
| Hydrolysed DNA | 0.002 |
| Hydrolysed RNA | 0.002 |
| Butylene glycol | 0.3 |
| Corosolic acid (from Eriobotrya Japonica Leaf Extract) | 0.1 |
| Sodium hydroxide | 0.07 |
| Salicylic acid | 0.001 |
| Glycogen | 0.001 |
| Serine | 0.1 |
| Threonine | 0.1 |
| Propylene glycol | 0.3 |
| Phosphatidylcholine | 0.2 |
| Hematite extract | 0.1 |
| Artemisia extract | 0.05 |
| Silanediol salicylate | 0.04 |

### Placebo formulation (w/w)

| | |
|---|---|
| Denatured alcohol | 51.6 |
| Water | q.s. 100 |
| Disodium EDTA | 0.05 |
| Sodium hydroxide | 0.07 |

### 1.4. Subjects participating in the trial

The volunteers participating in the trial were selected by the dermatologist from a panel of healthy male volunteers by applying the inclusion and exclusion criteria provided below.

### 1.4.1 Inclusion criteria

- Healthy male volunteers
- Age: between 20 and 55 years of age
- Race: Caucasian
- Volunteers with a clinical picture characterised by androgenic alopecia grade II - IV (Three volunteers per group)
- Volunteers who have not had anti-hair loss treatment in the 6 months preceding the study
- Undertaking not to use, in the course of the study, topical and/or systemic products with activity comparable to that of the product under investigation
- Undertaking not to change the normal daily routine

### 1.4.2 Exclusion criteria

- Volunteers who do not meet the inclusion criteria
- Previous history of allergy or sensitivity to cosmetic products, toiletries, sun products and/or
   to topical medications
- Diseases of the scalp
- Volunteers with dermatological problems in the test area
- Volunteers under local or systemic drug treatment
- Positive history of atopy.

### 1.5 Treatment arms

Following recruitment, the volunteers were subdivided into two groups of 23. The volunteers were included in the active-treatment or placebo group on the basis of their number of entry into the study. The two active and placebo treatment arms were randomised according to an appropriate statistical procedure (Efron's biased coin design) by the Study Director.

### 1.6 Conduct of the trial

Following recruitment, the dermatologist evaluated the baseline conditions (T0) of the parameters of interest. The instructions for use of the products were explained to the volunteers and they were given the study fact sheet together with the relevant products. The subsequent check-ups were then set at 2 (T2) and 4(T4) months of treatment. The parameters assessed are set out in the paragraphs below. With a view to standardising cosmetic habits relating to hair-washing, all the volunteers used the same shampoo (White plastic container. Anonymous, unlabelled).

### 1.7 Materials and Methods

The materials and the methods used in the course of the study are described below.

### 1.7.1 Evaluation of the grade of alopecia

The grade of alopecia was evaluated by means of the Hamilton - Norwood scale (see box 1 on the next page).

### 1.8 Phototrichogram

An area (of transition between thinning and balding) of 1.8 cm² was selected for shaving. The hair shaved inside the area was dyed so as to increase the contrast of grey or light-coloured hair. A photograph was then taken immediately after shaving and then after 2 days. These two photographs were then analysed by means of an image-analysis system able to recognise the individual hair fibres within the image. From comparison of the two images, the software is able to recognise hair that is in the growth phase (anagen phase) and hair that is not in the growth phase (telogen phase).

### 1.8.1 Pull test

The pull test is a diagnostic test useful for evaluating diffuse hair loss. Light traction is exerted on a lock of hair (around 60) in three areas (frontal, temporal and occipital) and the number of hairs extracted is counted. If at least 3 hairs are extracted by each pull or if more than 10 hairs are extracted overall in the three areas, the pull test is positive and indicative of telogen effluvium.

### 1.8.2 Digital macrophotography

Two photos were acquired per volunteer: 1 frontal (temples) and 1 at the level of the vertex.

### 1.8.3 Self-evaluation

After using the product for 2/4 months, the volunteers were asked to express their personal opinion by answering the questions in a questionnaire.

### 1.9 Results

On the basis of the results of the trial it has been found that treatment with Lotion 1 in volunteers with grade II, III, IV alopecia has proved effective in assisting the physiological hair growth. The results achieved are statistically significant.

### 1.9.1 After 2 months of treatment

Compared to the initial evaluation (T0), 2 months after treatment the phototrichogram recorded an 11.5% increase in hair in the growth phase (anagen) in 95.7% of volunteers participating in the study, with a -18.6% reduction of hair in the shedding phase (telogen).

In addition, in 89.5% of participating volunteers, the capacity of the hair to remain attached to its follicle following traction (resistance to traction - pull test) increased by 29.9%.

### 1.9.2 After 4 months of treatment

On the basis of the results of treatment with lotion 1, after 4 months an appreciable action on physiological hair growth was found in volunteers with grade II, III and IV alopecia.

Compared to the initial evaluation (T0), 4 months after treatment the phototrichogram recorded an 18.2% increase in hair in the growth phase (anagen) in 100% of volunteers participating in the trial, with a -29.1% reduction of hair in the shedding phase (telogen).

The changes recorded with the use of Lotion 1 are statistically significant in all investigational times monitored. The changes in the parameters analysed for cosmetic lotion 1 were all greater than in the placebo lotion, at all investigation times monitored.

## Claims

1. A composition for activating hair follicle stem cells to stimulate hair growth comprising the synergistic association of
i) a biologically active component that promotes the expression of marker proteins associated with the growth and proliferation of follicle stem cells, in an effective amount and
ii) a biologically active component that stimulates the proliferation of dermal papilla mesenchymal cells, in an effective amount, and a cosmetically acceptable carrier,
said composition being **characterised in that** the biologically active component i) comprises a peptide rice extract and the biologically active component ii) comprises corosolic acid.

2. Composition according to claim 1, wherein the biologically active component ii) comprising corosolic acid is a Eriobotrya japonica plant extract.

3. Composition according to claim 1 or 2, further comprising a biologically active component iii) that inhibits the 5-alpha reductase enzyme.

4. Composition according to claim 3, wherein the component iii) that inhibits the 5-alpha reductase enzyme comprises Biochanin-A.

5. Composition according to claim 4, wherein the component iii) that comprises Biochanin-A is a Trifolium pratense extract.

6. Composition according to any one of claims 1-5, further comprising a nicotinic acid ester.

7. Composition according to any one of claims 1-6, wherein said carrier is water or a hydroalcoholic solution.

8. Composition according to any one of claims 1-7, having the following formulation
- peptide rice extract, from 0.0001 to 10% by weight,
- corosolic acid or Eriobotrya japonica plant extract, from 0.0001 to 10% by weight,
- Biochanin-A or Trifolium pratense plant extract of 0.0001 to 10% by weight,
- water or hydroalcoholic solution to reach 100% by weight.

9. Composition according to any one of claims 1-8 in the form of trichological lotion for topical application or in the form of shampoo for the cleansing and simultaneous trichological treatment of the scalp.

10. Cosmetic use of a composition according to any one of claims 1-9 to stimulate the activity of hair follicle stem cells and/or hair growth.

## Patentansprüche

1. Zusammensetzung zum Aktivieren von Haarfollikelstammzellen zum Stimulieren des Haarwachstums, wobei die Zusammensetzung die synergistische Vereinigung
i) einer biologisch aktiven Komponente, die die Expression von Markerproteinen fördert, die dem Wachstum und der Vermehrung von Follikelstammzellen zugeordnet sind, in einer wirksamen Menge, und
ii) einer biologisch aktiven Komponente, die die Vermehrung von Hautpapillen-Mesenchymzellen stimuliert, in einer wirksamen Menge, und eines kosmetisch verträglichen Trägers umfasst,
wobei die genannte Zusammensetzung **dadurch gekennzeichnet ist, dass** die biologisch aktive Komponente i) einen peptidischen Reisextrakt umfasst und dass die biologisch aktive Komponente ii) Korosolsäure umfasst.

2. Zusammensetzung gemäß Anspruch 1, wobei die biologisch aktive Komponente ii), die Korosolsäure umfasst, ein Wollmispelpflanzenextrakt ist.

3. Zusammensetzung gemäß Anspruch 1 oder 2, die ferner eine biologisch aktive Komponente iii) umfasst, die das 5-Alpha-Reduktaseenzym hemmt.

4. Zusammensetzung gemäß Anspruch 3, wobei die Komponente iii), die das 5-Alpha-Reduktaseenzym hemmt, Biochanin A umfasst.

5. Zusammensetzung gemäß Anspruch 4, wobei die Komponente iii), die Biochanin A umfasst, einen Wiesenkleeextrakt umfasst.

6. Zusammensetzung gemäß einem der Ansprüche 1-5, die ferner ein Nikotinsäureester umfasst.

7. Zusammensetzung gemäß einem der Ansprüche 1-6, wobei der genannte Träger Wasser oder eine hydroalkoholische Lösung ist.

8. Zusammensetzung gemäß einem der Ansprüche 1-7 mit der folgenden Formulierung:
- peptidischer Reisextrakt von 0,0001 bis 10 Gew.-%,
- Korosolsäure oder Wollmispelpflanzenextrakt von 0,0001 bis 10 Gew.-%,
- Biochanin A oder Wiesenkleepflanzenextrakt von 0,0001 bis 10 Gew.-%,
- Wasser oder eine hydroalkoholische Lösung zum Erreichen von 100 Gew.-%.

9. Zusammensetzung gemäß einem der Ansprüche 1-8, in Form einer Haarlotion für die örtliche Anwendung oder in Form eines Shampoos für die Reinigung und gleichzeitige Haarbehandlung der Kopfhaut.

10. Kosmetische Verwendung einer Zusammensetzung gemäß einem der Ansprüche 1-9 zum Stimulieren der Aktivität von Haarfollikelstammzellen und/oder des Haarwachstums.

## Revendications

1. Composition destinée à activer des cellules souches de follicules pileux pour stimuler la croissance des cheveux, comprenant l'association synergique
i) d'un composant biologiquement actif qui favorise l'expression de protéines de marquage associées à la croissance et à la prolifération de cellules souches de follicules, en une quantité efficace, et
ii) d'un composant biologiquement actif qui stimule la prolifération de cellules mésenchymateuses de papilles dermiques, en une quantité efficace, et d'un support cosmétiquement acceptable,
ladite composition étant **caractérisée en ce que** le composant biologiquement actif i) comprend un extrait peptidique de riz et le composant biologiquement actif ii) comprend de l'acide corosolique.

2. Composition selon la revendication 1, dans laquelle le composant biologiquement actif ii) comprenant de l'acide corosolique est un extrait végétal d'Eriobotrya japonica.

3. Composition selon la revendication 1 ou 2, comprenant en outre un composant biologiquement actif iii) qui inhibe l'enzyme 5-alpha-réductase.

4. Composition selon la revendication 3, dans laquelle le composant iii) qui inhibe l'enzyme 5-alpha-réductase comprend de la Biochanine-A.

5. Composition selon la revendication 4, dans laquelle le composant iii) qui comprend de la Biochanine-A est un extrait de Trifolium pratense.

6. Composition selon l'une quelconque des revendications 1 à 5, comprenant en outre un ester d'acide nicotinique.

7. Composition selon l'une quelconque des revendications 1 à 6, dans laquelle ledit support est de l'eau ou une solution hydroalcoolique.

8. Composition selon l'une quelconque des revendications 1 à 7, ayant la formulation suivante :
- extrait peptidique de riz, de 0,0001 à 10 % en poids,
- acide corosolique ou extrait végétal d'Eriobotrya japonica, de 0,0001 à 10 % en poids,
- Biochanine-A ou extrait végétal de Trifolium pratense, de 0,0001 à 10 % en poids,
- de l'eau ou une solution hydroalcoolique pour atteindre 100 % en poids.

9. Composition selon l'une quelconque des revendications 1 à 8 sous forme de lotion trichologique pour une application topique, ou sous forme de shampooing pour le nettoyage et le traitement trichologique simultané du cuir chevelu.

10. Utilisation cosmétique d'une composition selon l'une quelconque des revendications 1 à 9 pour stimuler l'activité de cellules souches de follicules pileux et/ou la croissance des cheveux.
